# EUROPEAN PATENT APPLICATION

(11) **EP 4 166 569 A1**
(43) Date of publication of application: **19.04.2023**
(21) Application number: 21425051.6
(22) Date of filing: 18.10.2021
(51) Int. Cl.: C07K 16/28

(54) **ANTIBODIES DIRECTED AGAINST ALPHA-FOLATE RECEPTOR**

(71) Applicant: Fondazione IRCCS Istituto Nazionale dei Tumori, 20133 Milan (IT)
(72) Inventor: Figini, Mariangela, 20129 Milano (IT); Canevari, Silvana, 20054 Segrate (MI) (IT)
(74) Representative: Gerli, Paolo

(57) **Abstract**

New antibodies are described having high specificity to the α-folate receptor, being of medical interest in the therapy of tumours and of other diseases involving an increased expression of the α-folate receptor. All the antibodies of the present invention share the same six specific CDR in the V_{H} and V_{L} regions, herein defined by SEQ.ID.Nos: 1-6, responsible for the α-folate receptor specificity. In one embodiment, a subgroup of these antibodies identified as A-type is further characterized by an extended duration of binding to the α-folate receptor and, in an additional embodiment, another subgroup involving some aminoacidic modifications and identified as B-type is also characterized by an increased compatibility for human therapy.

## Description

### FIELD OF THE INVENTION

The invention relates to the field of antibodies for use in human therapy, in particular being directed to the a-folate receptor (FRa), with utility in the treatment of tumours and other diseases related to the activity and expression of this receptor.

### STATE OF THE ART

Folate receptor a (FRa) came into focus as an anticancer target many decades after the successful development of drugs targeting intracellular folate metabolism, such as methotrexate and pemetrexed. Binding to FRα is one of several methods by which folate is taken up by cells; this receptor is an attractive anticancer drug target owing to its overexpression in a range of solid tumours, including ovarian, lung and breast cancers. Furthermore, using FRα to better localize effective anticancer therapies to their target tumours using platforms such as antibody-drug conjugates, small-molecule drug conjugates, radioimmunoconjugates and, more recently, chimeric antigen receptor T cells could further improve the outcomes of patients with FRα-overexpressing cancers (Scaranti et al Nat Rev Clin Oncol 2020 Jun;17(6):349-359). FRa can also be harnessed for predictive biomarker research.

The FRa is a glycosylphosphatidyl-inositol (GPI)-linked protein and is overexpressed in more than 90% of epithelial ovarian carcinoma (EOC) and also in various tumors of mesodermic derivation, like pleural mesothelioma in which it is expressed in more than 50% of cases (Salazar M.D. et al., Cancer Metastasis Rev. 2007 Mar;26(1):141-52.). Although the function of FRa in cancers is not fully understood, its overexpression is associated with increased tumor aggressiveness (Toffoli G et al., Int J Cancer. 1998 Apr 17;79(2):121-6.; Elnakat H et al., Adv Drug Deliv Rev. 2004 Apr 29;56(8):1067-84) and might confer growth advantage by increasing folate availability to cancer cells (Bottero F et al., Cancer Res. 1993 Dec 1;53(23):5791-6).

Folate receptor a (FRα) is a valid anticancer drug target for multiple reasons. These reasons include: the limited expression in nonmalignant tissues coupled with overexpression in malignant tissues of certain cancers, making FRα a possible target using multiple platforms, such as antibodies, antibody- drug conjugates (ADC), bi- specific antibodies (Canevari S. et al Cancer Immunol Immunother. 1997 Nov-Dec;45(3-4):187-9) or chimeric antigen receptor (CAR) T cells (Song DG et al. Blood. 2012 Jan 19;119(3):696-706; Song et al. Cancer Res. 2011 Jul 1;71(13):4617-27) a high level of affinity for non- physiological folates, such as folic acid, making it targetable using folic acid conjugates; and, to a limited extent, its involvement in signal transduction and pro- survival signalling in cancer cells overexpressing FRa.

Three murine monoclonal antibodies (mAbs) were developed by two independent research groups, MOv18 and MOv19 (Miotti S et al., Int J Cancer. 1987 Mar 15;39(3):297-303) and LK26 (Garin-Chesa P et al., Am J Pathol. 1993 Feb;142(2):557-67) with high affinity to ovarian tumor cells. These antibodies and their derivatives are now used in many antibody-based trials. All these mAbs were directed against FRα but the epitope recognized by MOv18 is different from epitopes recognized by MOv19 and LK26. Concerning MOv18 and MOv19 initially they were evaluated for their ability to target radioisotopes at tumor level (Gadina M et al., Int J Rad Appl Instrum B. 1991;18(4):403-8.; Coliva A et al., Cancer Immunol Immunother. 2005 Dec;54(12):1200-13; Zacchetti A et al., Nucl Med Biol. 2009 Oct;36(7):759-70.) and later, several different derivatives of MOv18 and MOv19 were assessed in diverse therapeutic applications. The mAb LK26 was evaluated in preclinical studies for its in vivo anti-tumor activity in a subcutaneus xenograft model (Ebel W et al., Cancer Immun. 2007 Mar 9;7:6.), but did not enter clinical studies because of its murine origin. The major limitation in therapeutic use of murine mAbs is the immunogenicity of their protein sequence. Indeed, these mAbs can give rise to an immunogenic response referred to as human anti-mouse antibodies (HAMA) that reduce mAbs efficacy during following therapeutic application. A second limitation is the reduced therapeutic efficacy due to a relatively faster clearance in humans, potentially improved by the HAMA response. Third, murine mAbs present relatively weak effector functions, reduced ability to recruit effector cells or complement proteins when compared to human mAbs (Presta L.G., Adv Drug Deliv Rev. 2006 Aug 7;58(5-6):640-56). In order to overcome these limitations, chimeric mAbs were designed and though they exhibit less immunogenicity than murine mAbs the chimeric version can cause an anti-chimeric mAb response. Humanization is one of the methods that can lead to reduce the potential immunogenicity of therapeutic mAbs. Farletuzumab, the humanized version of LK26, a reagent with a high affinity to FRa similar to the original murine mAb is now in clinical trial as an ADC antibody. (Shimizu T et al. Clin Cancer Res. 2021 Jul 15;27(14):3905-3915)

### SUMMARY

A new group of monoclonal antibodies is now identified, having a high specificity to FRa, being useful in antitumour therapy, and for the treatment of other diseases involving an increased cell expression of FRa. All the antibodies of the invention are characterized by the following six complementarity-determining regions (CDR) located within their V_{H} or V_{L} chains, identified by the SEQ.ID.NO: 1-6. Exemplifying antibodies containing the six aforementioned CDR are defined as follows:
Antibody I), having V_{H} defined by SEQ.ID.NO:9 and V_{L} defined by SEQ.ID.NO:10; the corresponding encoding nucleotide sequences are provided herein as SEQ.ID.NO:13 and SEQ.ID.NO:14
Antibody II) having V_{H} is defined by SEQ.ID.NO: 11 and V_{L} is defined by SEQ.ID.NO: 12.

The monoclonal antibodies containing the above mentioned six CDRs show specificity to FRa: their peptide structures and the nucleotide sequences encoding for them have never been disclosed in the art. A preferred subgroup of the present monoclonal antibodies, herein referred as "A-type", shows the additional advantage of a time-extended binding to the FRα: this feature, which is different and additional to the CDR-driven receptor specificity (i.e. the capacity to distinguish the target cells from non-target cells) denotes an unexpectedly high binding strength; this improvement has been obtained by introducing specific aminoacids in the V_{H} and V_{L} chains, in areas laying outside the aforementioned CDRs, i.e. without modifying the CRDs.

In a development, the A-type antibodies have been further modified in other parts of the V_{H} and/or V_{L} chains lying outside the aforementioned CDRs, i.e. without modifying the CDRs, obtaining an improved compatibility for human therapy; this sub-group of antibodies is defined herein as "B-type".

The high specificity and the improved kinetic of binding of the antibodies reported herein results with the possibility to use the variable domains as a monovalent antibody fragment, such as the single chain form (scFv). If the recruitment of the effector cells is not necessary, the use of scFvs, due to their reduced size, is ideally suited to carry an anti-tumour drug of interest as they can better penetrate the tumour to reach their target (herein the FRa expressed on the surface of tumour cells). Nevertheless, the present invention is not limited to single chain antibodies but extends also the corresponding variable domain antibodies in the standard immunoglobulin: these are expected to have even higher specificity to the FRa due to their bivalent character.

The invention extends to the aforementioned monoclonal antibodies for use in therapy and, in particular, for the treatment of tumour as well as other diseases involving an increased expression of FRα. The invention also includes pharmaceutical compositions comprising a monoclonal antibody as herein described.

### DESCRIPTION OF THE FIGURES

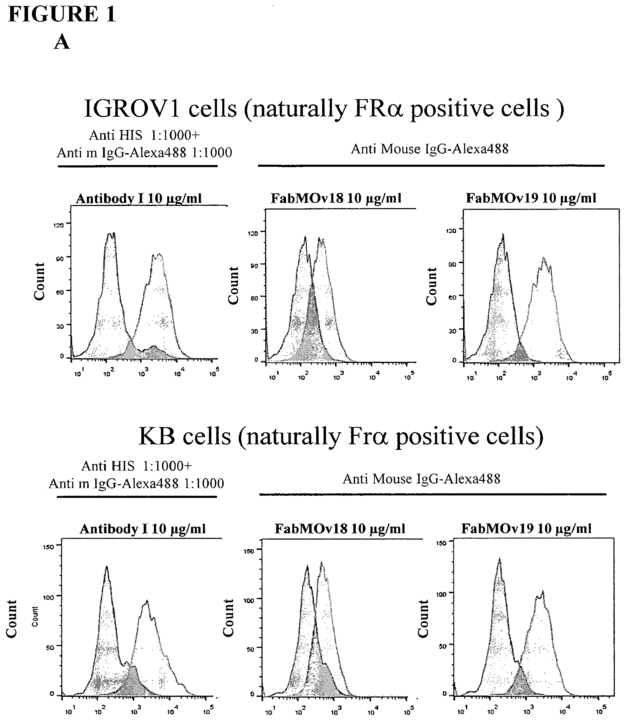

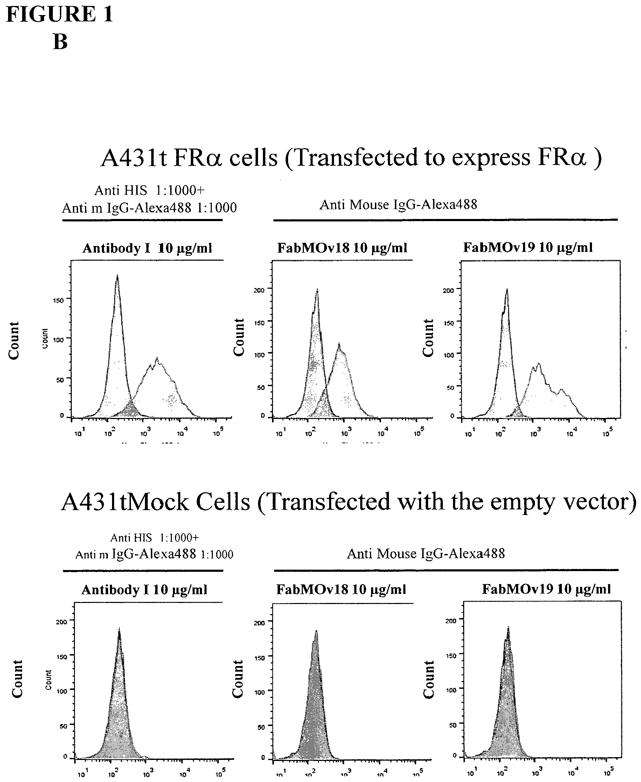

Figure 1 (A-B): results from FACS studies on Antibody I, FAb-MOv18, FAb-MOv 19.
Figure 2: results from Biacore binding studies on Antibody I and FAb-MOv18. Kinetic on FRa of: (A) Antibody I AND (B) Monovalent MOv18 at dilutions from 50 to 0.4nM. (C) Comparison of the binding of Antibody I (blue lane) and FAb-MOv18 (green lane) at 200nM concentration

### DETAILED DESCRIPTION OF THE INVENTION

In accordance with standard knowledge, the term "monoclonal antibody" refers herein to a high molecular weight peptide, possibly with a three-dimensional immunoglobulin structure, having specificity to a specific antigenic target. The antigenic target of interest for the present antibodies is the FRa. The present antibodies include a variable region (Fv) containing the variable high molecular weight chain (V_{H}) and the variable low molecular weight chain (V_{L}). They may also include a constant region containing the constant high/low molecular weight chains (C_{H}/C_{L}), structured and positioned according to standard antibody conformation. In this description, the numbering of aminoacids making up the respective peptide chains is a standard progressive one, i.e. ranging from 1 to *n* for a peptide chain containing *n* aminoacids.

The antibodies of the invention are characterized herein by their variable region part, which is responsible for the specificity to the FRα. They are generally defined by their six CRDs contained in their V_{H} and V_{L} regions, identified by the following SEQ.ID.NO: 1-6.
V_{H} CDR1, defined by SEQ.ID.NO: 1;
V_{H} CDR2, defined by SEQ.ID.NO: 2;
V_{H} CDR3, defined by SEQ.ID.NO: 3;
V_{L} CDR1, defined by SEQ.ID.NO: 4;
V_{L} CDR2, defined by SEQ.ID.NO: 5;
V_{L} CDR3, defined by SEQ.ID.NO: 6.

The V_{H} CDR1 is preferably located between antibody's V_{H} frameworks 1 and 2.

The V_{H} CDR2 is preferably located between antibody's V_{H} frameworks 2 and 3.

The V_{H} CDR3 is preferably located between antibody's V_{H} frameworks 3 and 4.

The V_{L} CDR1 is preferably located between antibody's V_{L} rameworks 1 and 2.

The V_{L} CDR2 is preferably located between antibody's V_{L} frameworks 2 and 3.

The V_{L} CDR3 is preferably located between antibody's V_{L} frameworks 3 and 4.

The above referred V_{H} frameworks 1, 2, 3 and V_{L} frameworks 1, 2, 3 are those defined according to the standard antibody structure of IMGT/Collier-de-Perles (Domain V): (Ruiz M, et al. Immunogenetics. 2002).

All said CDRs are constant in structure and simultaneously present in the antibodies of the invention.

The present monoclonal antibodies can have a single chain or a classical immunoglobulin structure, e.g. containing the variable domains transformed in the classical IgG format: when in the single chain conformation, the six CRDs will be present once; if it is in the classical immunoglobulin structure, they will be present twice.

The monoclonal antibodies defined by the six CRD's represent the main embodiment of the present invention. These antibodies do not include the antibody Mov18 which is characterized by V_{H} defined by SEQ.ID.NO: 7 and V_{L} defined by SEQ.ID.NO: 8.

A sub-group of the present antibodies, containing particular aminoacids at certain positions of the V_{H} and V_{L} chains external to said CDRs and here defined as "A-type", have shown the additional unexpected property of a prolonged binding to the FRa. These A-type antibodies are characterized in that the V_{H} contains 120 aminoacids and meet at least ten of the conditions according to

**Table 1:**

| aminoacid | at position |
|---|---|
| V | 5 |
| S | 9 |
| K | 12 |
| K | 13 |
| V | 20 |
| R | 38 |
| A | 40 |
| M | 48 |
| V | 76 |
| L | 81 |
| Q | 82 |
| I | 83 |
| S | 84 |
| K | 87 |
| A | 88 |
| T | 91 |
| T | 116 |
| S | 120 |

and, at the same time, V_{L} contains 107 aminoacids and meets at least eight of the conditions according to:

**Table 2**

| aminoacid | at position |
|---|---|
| S | 7 |
| P | 8 |
| V | 15 |
| T | 22 |
| G | 41 |
| K | 42 |
| A | 43 |
| P | 44 |
| T | 72 |
| F | 73 |
| S | 76 |
| S | 77 |
| Q | 79 |
| P | 80 |
| T | 85 |

In preferred variants, the A-type antibodies meet a higher number of conditions of Tables 1 and 2, in particular:
the V_{H} meets at least fourteen of the conditions of Table 1 and the V_{L} meets at least eleven of the conditions of Table 2, or
the V_{H} meets at least fifteen of the conditions of Table 1 and the V_{L} meets at least twelve of the conditions of Table 2, or
the V_{H} meets at least sixteen of the conditions of Table 1 and the V_{L} meets at least thirteen of the conditions of Table 2, or
the V_{H} meets at least seventeen of the conditions of Table 1 and the V_{L} meets at least fourteen of the conditions of Table 2, or
the V_{H} meets all conditions of Table 1 and the V_{L} meets all conditions of Table 2.

A preferred antibody meeting these criteria, here referred as Antibody I, is characterized by the SEQ.ID.NO: 9 (V_{H}) and SEQ.ID.NO: 10 (V_{L}).

When introduced in the present antibodies, the A-type modifications also confer to the antibody an increased degree of compatibility with the human organism, thus involving low or no risk of generating autoimmune reactions when used in human therapy.

In a preferred embodiment, the A-type antibodies are further modified in the non-CDR regions of their V_{H} and V_{L} chains obtaining an even higher degree of human compatibility. This subgroup of antibodies, herein defined of "B-type" contains the following modifications:
in the V_{H}:

**Table 3**

| aminoacid | at position |
|---|---|
| V | 11 |
| Q | 65 |
| R | 67 |
| E | 82 |

and/or in the V_{L:}

**Table 4**

| aminoacid | at position |
|---|---|
| Q | 24 |
| F | 71 |

Preferably the B-type antibodies include both the modifications of Table 3 and of table 4. A preferred antibody meeting these criteria, here referred as Antibody II, is characterized by the SEQ.ID.NO: 11 (V_{H}) and SEQ.ID.NO: 12 (V_{L}).

A further object of the present invention is the provision of the aforementioned monoclonal antibodies for use in therapy and, in particular, for the treatment of tumour as well as any other disease involving an increased expression of FRa. Preferred targeted tumours are ovarian carcinoma (e.g. epithelial ovarian carcinoma (EOC)) and tumours of mesodermic derivation (e.g. pleural mesothelioma). Further non-limitative targeted tumours are non-mucinous adenocarcinomas of the ovary, uterus and cervix, testicular choriocarcinoma, trophoblastoma, ependymal brain tumors, laryngeal epithelial tumour, pituitary adenocarcinoma, colon, renal, breast, lung and kidney tumours, etc.

A further object of the invention is a conjugate of the present antibodies with a substrate therapeutically active on diseases involving an increased expression of FRa; the conjugate is useful for the targeted delivery of the substrate to the FRa; as to the possible substrate, there is no limitation: it can be e.g. a drug molecule, a cell or cell component, a nanoparticle, etc.; typically, the drug molecule is an antitumour drug molecule.

The invention also includes pharmaceutical compositions comprising an antibody or conjugate thereof as herein described.

The invention and its effects are now described by means of the following non-limitative examples.

### EXPERIMENTALS

### 1. FACS ANALYSIS

Monovalent forms (Fabs) of the primary antibodies MOv18 (SEQ.ID.NOs:7-8) or MOv19, and Antibody I (ScFv SEQ.ID.NOs:9-10) (10 µg/ml) were added to tumor cell (5x105) in 100 µl PBS+ 0.03% BSA, and the mixture was incubated for 30 min on ice. The binding was detected by secondary antibody anti HIS Tag (produced in mouse) for 30 min on ice. All antibodies binding was revealed with an Alexa488 conjugated anti mouse antibody for 30 min on ice. For each sample 5000 cells were analysed with FACS Canto using DIVA software. The score was calculated as the ratio between mean fluorescence of the sample and mean fluorescence of the secondary antibody alone. The results are presented in Figure I: it can be seen that all the tested antibodies show specificity for the FRa.

### 2. BIACORE BINDING STUDIES

Biacore is an instrument based on Surface Plasmo Resonance for the characterization of biomolecular interactions (Jason-Moller L et al Curr Protoc Protein Sci. 2006 Sep; Chapter 19: Unit 19.13), in this case antibodies and FRa. MOv18 Fab (SEQ.ID.NOs:7-8) and Antibody I (ScFv, SEQ.ID.NOs:9-10) were tested. Kinetic analyses were performed with BIACORE T200 equipment. 400 RU of FRa was covalently bound to a CM 5 sensor chip using the amine coupling kit, using an antigen concentration of 5 µg/ml in 10 mM sodium Acetate. Residual activated groups were blocked by injection of 1.0 M ethanolamine pH 8.5. Different concentration, from 50 to 0.4 nM, of Antibody I or Fab' MOv18 were analyzed at a flow rate of 30 µl/min and dissociation was allowed to proceed for at least 5 min to determine the binding kinetics. Residual binding was detached using 10 mM glycine-HCl pH 2.7. The respective affinities were deduced from the binding curve using Biacore T200 evaluation software.

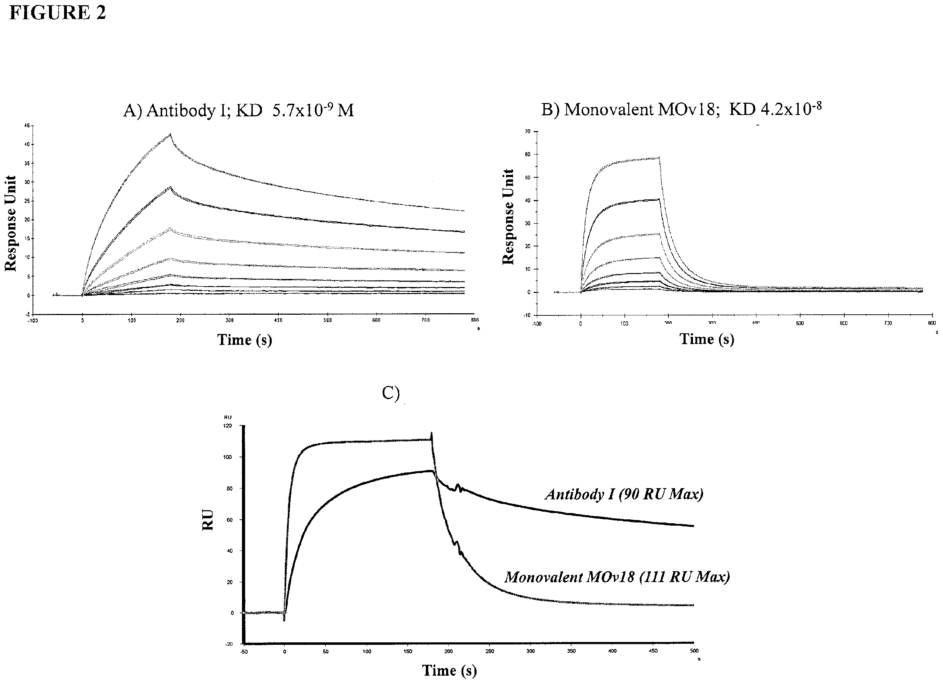

The results are presented in Figure 2: after 180 sec. of association, Antibody I presented a much slower dissociation kinetics as compared to MOv18 FAb. In particular, while at time 300 sec the binding of Antibody I was still about 75% of the plateau, the binding of MOv18 FAb was substantially nil. Further interestingly, after time 300 sec, the binding of Antibody I reached a steady state at levels still comparable to the plateau, pointing to a much stronger and almost permanent binding capacity for antibody I, not at all present for MOv18 FAb. It is thus shown that, although both antibodies share useful FRa specificity, Antibody I showed a much higher binding strength to this receptor, which makes it an improved candidate for the treatment of diseases characterized by the enhanced expression of this receptor.

### 3. HUMANIZATION

Modifications were introduced in Antibody I in order to increase human compatibility. The modification
V_{H}: 11-V; 65-Q; 67-R; 82-E;
V_{L}: 24-Q; 71-F;
resulting in Antibody II (SEQ.ID.NOs: 11-12) proved optimal in increasing compatibility measured according to "Humanization of antibodies using a machine learning approach on large-scale repertoire data" (Marks C et al Bioinformatics. 2021 Jun 10:btab434) while maintaining the structural conformation of the antibody and its consequent binding profile.

### SEQUENCE LISTINGS

SEQ.ID.NO:1 V_{H} CDR1
   DYIFTNYD
SEQ.ID.NO:2 V_{H} CDR2
   IDPRSGKS
SEQ.ID.NO:3 V_{H} CDR3
   ATMYYYGSSPPMDY
SEQ.ID.NO:4 V_{L} CDR1
   QDINNF
SEQ.ID.NO:5 V_{L} CDR2
   YTS
SEQ.ID.NO:6 V_{L} CDR3
   QQSSTIPRT
SEQ.ID.NO:7 V_{H} Mov18 
QVQLQQSGAELARPGASVKLSCKASDYIFTNYDITWVKQRPGQGLEWIGEI
DPRSGKSYYNEKFKGKSTLTADKSSSTAYMELRSLTSEDSAVYFCATMYYY
GSSPPMDYWGQGTSVTVSRRE
SEQ.ID.NO:8 V_{L} Mov18 
DIQMTQTTSSLSASLGDRVTISCRASQDINNFLNWYQQKPDGTVKLLIYYTS
RLHSGVPSRFSGSGSGTDYSLTIINLEQEDIAIYFCQQSSTIPRTFGQGTKLE
IK
SEQ.ID.NO:9 V_{H} Antibody I 
QVQLVQSGSELKKPGASVKVSCKASDYIFTNYDITWVRQAPGQGLEWMGE
IDPRSGKSYYNEKFKGKSTLTADKSVSTAYLQISSLKAEDTAVYFCATMYYY
GSSPPMDYWGQGTTVTVS
SEQ.ID.NO:10 V_{L} Antibody I 
DIQMTQSPSSLSASVGDRVTITCRASQDINNFLNWYQQKPGKAPKLLIYYTS
RLHSGVPSRFSGSGSGTDYTFTISSLQPEDIATYFCQQSSTIPRTFGQGTKL
EIK
SEQ.ID.NO: 11 V_{H} Antibody II 
QVQLVQSGSEVKKPGASVKVSCKASDYIFTNYDITWVRQAPGQGLEWMG
EIDPRSGKSYYNEKFQGRSTLTADKSVSTAYLEISSLKAEDTAVYFCATMYY
YGSSPPMDYWGQGTTVTVS
SEQ.ID.NO: 12 V_{L} Antibody II 
DIQMTQSPSSLSASVGDRVTITCQASQDINNFLNWYQQKPGKAPKLLIYYTS
RLHSGVPSRFSGSGSGTDFTFTISSLQPEDIATYFCQQSSTIPRTFGQGTKL
EIK
SEQ.ID.NO:13 V_{H} Antibody I (encoding nucleotide sequence) 
CAGGTGCAGCTGGTGCAATCTGGGTCTGAGTTGAAGAAGCCTGGGGCC
TCAGTGAAAGTTTCCTGCAAGGCTAGCGACTACATCTTCACTAACTATGA
CATAACCTGGGTGCGACAGGCCCCTGGCCAGGGGCTTGAGTGGATGGG
AGAGATTGATCCTAGGAGTGGTAAGAGTTATTATAATGAGAAATTCAAGG
GCAAGTCCACACTGACTGCAGACAAATCTGTCAGCACGGCATATCTGCA
GATCAGCAGCCTAAAGGCTGAGGACACGGCCGTGTATTTCTGTGCGACA
ATGTACTACTACGGCTCGAGCCCCCCTATGGACTACTGGGGTCAAGGAA
CCACTGTCACCGTCTCC
SEQ.ID.NO: 14 V_{L} Antibody I (encoding nucleotide sequence)

GATATCCAGATGACCCAGAGTCCAAGCAGCCTGAGTGCTAGCGTGGGA
GATCGCGTGACCATCACGTGTAGGGCCAGTCAGGACATTAACAATTTTTT
AAACTGGTACCAGCAGAAGCCTGGTAAGGCTCCAAAGCTGCTGATCTAC
TACACCTCGAGATTACACTCAGGAGTGCCATCTCGCTTCAGCGGTTCTG
GAAGCGGTACCGACTATACCTTCACCATCAGCAGCCTCCAGCCTGAGGA
TATCGCTACGTACTTCTGTCAACAGAGTAGTACGATTCCTAGGACGTTCG
GACAGGGAACCAAGCTGGAAATCAAG

## Claims

1. Monoclonal antibody directed against the alpha folate receptor (FRα), **characterized by** comprising the following CDRs:
V_{H} CDR1, defined by SEQ.ID.NO: 1,
V_{H} CDR2, defined by SEQ.ID.NO: 2,
V_{H} CDR3, defined by SEQ.ID.NO: 3,
V_{L} CDR1, defined by SEQ.ID.NO: 4,
V_{L} CDR2, defined by SEQ.ID.NO: 5, and
V_{L} CDR3, defined by SEQ.ID.NO: 6,
excluding an antibody having V_{H} defined by SEQ.ID.NO: 7 and V_{L} defined by SEQ.ID.NO: 8.

2. Monoclonal antibody according to claim 1, wherein V_{H} contains 120 aminoacids and further meets at least ten of the conditions according to
**Table 1:**
| aminoacid | at position |
|---|---|
| V | 5 |
| S | 9 |
| K | 12 |
| K | 13 |
| V | 20 |
| R | 38 |
| A | 40 |
| M | 48 |
| V | 76 |
| L | 81 |
| Q | 82 |
| I | 83 |
| s | 84 |
| K | 87 |
| A | 88 |
| T | 91 |
| T | 116 |
| s | 120 |
and V_{L} contains 107 aminoacids and further meets at least eight of the conditions according to:
**Table 2**
| aminoacid | at position |
|---|---|
| S | 7 |
| P | 8 |
| V | 15 |
| T | 22 |
| G | 41 |
| K | 42 |
| A | 43 |
| P | 44 |
| T | 72 |
| F | 73 |
| s | 76 |
| s | 77 |
| Q | 79 |
| P | 80 |
| T | 85 |

3. Antibody according to claim 2, wherein the V_{H} meets at least fourteen of the conditions of Table 1 and the V_{L} meets at least eleven of the conditions of Table 2.

4. Antibody according to claims 2-3, with improved compatibility for human therapy, comprising the following additional conditions in the V_{H}:
| aminoacid | at position |
|---|---|
| V | 11 |
| Q | 65 |
| R | 67 |
| E | 82 |

5. Antibody according to claims 2-3, with improved compatibility for human therapy, comprising the following additional conditions in the V_{L:}
| amino acid | at position |
|---|---|
| Q | 24 |
| F | 71 |

6. Antibody with improved compatibility for human therapy, in accordance with both claims 4 and 5.

7. Antibody according to claim 3, wherein V_{H} is defined by SEQ.ID.NO:9 and V_{L} is defined by SEQ.ID.NO: 10.

8. Antibody according to claims 6, wherein V_{H} is defined by SEQ.ID.NO: 11 and V_{L} is defined by SEQ.ID.NO: 12.

9. Antibody according to claims 1-8 being a single chain antibody.

10. Antibody according to claims 1-9, in conjugated form with a substrate active on tumour or other diseases **characterized by** an increased cell expression of FRα.

11. Pharmaceutical composition comprising an antibody as defined in claims 1-10.

12. Antibody as defined in claims 1-10 for use in therapy.

13. Antibody as defined in claims 1-10 for use in the treatment of tumour or other diseases **characterized by** an increased cell expression of FRα.
